# EUROPEAN PATENT APPLICATION

(11) **EP 3 928 802 A1**
(43) Date of publication of application: **29.12.2021**
(21) Application number: 20181316.9
(22) Date of filing: 22.06.2020
(51) Int. Cl.: A61L 2/07, A61L 2/26

(54) **STEAM STERILIZER**

(71) Applicant: Koninklijke Philips N.V., 5656 AG Eindhoven (NL)
(72) Inventor: LUI, Kwan Fai, 5656 AE Eindhoven (NL); BIJLOO, Michiel Dirk Augustinus, 5656 AE Eindhoven (NL); VAN DER ZWAN, Eduard Antonius, 5656 AE Eindhoven (NL); STOLK, Theodoor, 5656 AE Eindhoven (NL)
(74) Representative: Philips Intellectual Property & Standards

(57) **Abstract**

The invention proposes a steam sterilizer comprising a base (10), a basket (20), a bottom plate (30) and a collector (60). The base comprises a heating unit (101) and water container (102), the heating unit is arranged to heat water contained in the water container to generate steam. The basket (20) is positioned on the base (10), the basket has an open area (201) at the bottom to let steam into the basket. The bottom plate (30) is positioned in the basket for holding articles to be sterilized such as feeding bottles or other baby items; and the collector (60) is positioned under the bottom plate for collecting fluid dripping down from the bottom plate. By this way, the fluid is prevented from dripping down to the water container.

## Description

### FIELD OF THE INVENTION

The present invention relates to a sterilizer for sterilizing feeding bottles and/or other baby items. In particular, the present invention relates to a sterilizer for sterilizing feeding bottles and/or other baby items with steam.

### BACKGROUND OF THE INVENTION

Conventional sterilisers use hot steam to heat feeding bottles and teats to a high temperature to kill bacteria. These appliances usually use a heating element inside a water container to boil water and convert it into steam.

CN205332691U discloses a sterilizer which comprises a base and a basket. The base comprises a heater under a water container to convert the water into steam. The basket comprises a bottom plate and an upper plate for holding the feeding bottles and teats respectively. The basket sits on the base so that the bottom plate is above the water container . The bottom plate and the upper plate have multiple holes to allow the steam to enter into the basket and sterilize the feeding bottles and teats.

### SUMMARY OF THE INVENTION

A problem with conventional sterilizers is that the inside of the water container can become spoiled with burn marks. This problem happens when a feeding bottle is not completely cleaned before a sterilization. The milk residue or unclean particles in the feeding bottle contaminates the steam that then condenses into water and flows back into the water container. Near the end of the sterilization cycle, the contaminated water tends to overheat, and create a burn mark and unpleasant smell. A hygiene problem can also develop due to the difficulty of cleaning this part of these appliances.

This invention proposes a sterilizer that comprises:
A base having a heating unit and a water container, the heating unit is arranged to heat water contained in the water container to generate steam;
A basket positioned on the base, the basket having an open area at the bottom to let steam enter into the basket;
A bottom plate positioned in the basket for holding articles to be sterilized;
A collector positioned under the bottom plate for collecting fluid dripping down from the bottom plate.

The collector collects the water that condenses from the steam to ensure that water contaminated with milk or particles cannot return to the heating surface, i.e the water container. Thus, this invention overcomes the problem of the burn marks.

Advantageously, the collector may be attached to whichever of the basket, the bottom plate, or the base.

Advantegeously, the collector may be detached from to whichever of the basket, the bottom plate, or the base.

Advantageously, the collector may be a groove that comprises an annular bottom, a first sidewall and a second sidewall. The first sidewall surrounds the outer edge of the annular bottom of the collector. The second sidewall surrounds the inner edge of the annular bottom of the collector.

Advantageously, the collector is part of the basket, wherein the sidewall of the basket forms the first sidewall of the collector, the sidewall of the basket extends inwards along the radial direction to form the bottom of the collector and further extends upwards to form the second sidewall of the collector. The second sidewall of the collector surrounding the open area of the basket and the second sidewall of the collector is lower than the lower surface of the bottom plate.

Advantageously, the collector comprises a bottom and a sidewall surrounding the bottom, i.e like a bowl or a dish with a single enclosed sidewall. The bottom may be in any regular or irregular shape, round, oval, square, rectangular, polygon, etc. The top edge of the sidewall of the collector is lower than the lower surface of the bottom plate.

Advantageously, the bottom plate may be detachably or fixedly positioned in the basket.

Advantageously, the bottom plate comprises a first area with multiple holes allowing steam from the water container to pass through, wherein the horizontal projection of the collector covers the horizontal projection of the first area of the bottom plate. In this way, the condensed water and any contaminants can be collected by the collector.

It is to be understood that in the context of the invention the term "projection" means the representation of a line, figure, or solid on a given plane as it would be seen from a particular direction or in accordance with an accepted set of rules. The term "horizontal projection" means a projection made on a plane parallel to the horizontal plane, in other words, the "horizontal projection of an object" in the context of the invention means a representation of the object on a plane parallel to the horizonal plane as it would be seen from a vertical direction.

Advantageously, the bottom plate comprises a second area which is a solid plate without a hole. The horizontal projection of the second area covers the horizontal projection of the open area of the basket at the bottom of the basket.

Advantageously, the second area of the bottom plate is in the center and is surrounded by the first area of the bottom plate.

Advantageously, the first area of the bottom plate is in the center and is surrounded by the second area of the bottom plate.

Advantageously, the first area and the second area of the bottom plate are adjacent to each other with a boundary that is a non-closed straight line or curved line, i.e. neither the first area surrounds the second area nor the second area surrounds the first area.

Advantageously, among the multiple holes in the first area, there is a set of holes that extend upwards to form a set of hollowed pipes. The other holes do not extend upwards to form hollowed pipes and therefore are holes.

The feeding bottles to be sterilized may be put on the hollowed pipes so that the steam passing through the hollowed pipes sterilizes the insides of the bottles. Supporters hold the feeding bottles in place over the hollowed pipes.

In this embodiment, the total area of the outlet of the set of hollowed pipes is larger than the total area of the rest of the multiple holes without hollowed pipes. Preferably the area of the outlet of the set of the hollowed pipes is twice or more than twice larger than the total area of the rest of the multiple holes. By this area design, the resistance of the hollowed pipes is smaller than the rest of the multiple holes without hollowed pipes so that the steam will mainly pass through the hollowed pipes to sterilize the inside of the feeding bottle.

Advantageously, the diameter of the outlet of each of the hollowed pipes is smaller than 13 millimeters, while the bottle supporters of the feeding bottles have a height of more than 4 millimeters. This makes the resistance of each of the hollowed pipes that transports the steam into the feeding bottles more substantial than the resistance to the escape of steam from the feeding bottles. Thus, a hollowed pipe without a feeding bottle on it still provides substantial resistance to the steam entering the basket so that the steam does not mainly pass though hollowed pipes without feeding bottles over them, and instead passes evenly through all of the hollowed pipes.

The basket can also be used with a high end sterilizer, which has a drying cycle whereby after the sterialization cycle, the condesate in the collector is evaporated. Thus, the user does not need to empty the collector.

According to another aspect of the invention, a basket is proposed for use in a sterilizer which sterilizes articles using steam. The basket having an open area at the bottom allowing steam to enter into the basket, a bottom plate positioned in the basket for holding articles to be sterilized, wherein the basket comprises a collector for collecting fluid dripping down from the bottom plate. The collector may be a groove comprising an annular bottom, a first sidewall, and a second sidewall. The first sidewall of the collector surrounds the outer edge of the annular bottom of the collector, and the second sidewall of the collector surrounds the inner edge of the annular bottom of the collector. The sidewall of the basket forms the first sidewall of the collector, the sidewall of the basket extends inwards along the radial direction to form the bottom of the collector and further extends upwards to form the second sidewall of the collector.

According to another aspect of the invention, a bottom plate is proposed for use in a sterilizer which sterilizes articles using steam. The bottom plate being used for holding articles to be sterilized, the bottom plate being positioned in a basket, with a collector positioned under the bottom plate for collecting fluid dripping down from the bottom plate.

According to another aspect of the invention, a base is proposed for use in a sterilizer which sterilizes articles using steam. The base comprises a heating unit and a water container. The heating unit is arranged to heat water in the water container to generate steam to sterilize articles. The sterilizer comprises a basket positioned on the base, the basket having an open area at the bottom to let steam enter into the basket. The sterilizer further comprises a bottom plate positioned in the basket for holding articles to be sterilized. The base further comprises a collector for collecting fluid dripping down from the bottom plate.

In the proposed bottom plate and base, the collector may be a groove comprising of an annular bottom, a first sidewall and a second sidewall, wherein the first sidewall surrounds the outer edge of the annular bottom of the collector, and the second sidewall surrounds the inner edge of the annular bottom of the collector. Or the collector may comprise a bottom and a sidewall that surrounds the bottom.

According to the aspects and embodiments described above, the limitations of existing techniques are addressed. In particular, the above-described aspects and embodiments enable an improved sterilizer which can prevent the condensed water and contaminants from dripping into the water container and leaving burnt marks. Some embodiments described above further offer improved steam flow with better efficiency.

These and other aspects of the disclosure will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
FIG. 1 depicts a stereogram of a sterilizer according to an embodiment of the invention;
FIG.2 depicts the exploded view of the sterilizer according to the embodiment of FIG. 1;
FIG.3 depicts a sectional view of the sterilizer according to the embodiment of FIG. 1;
FIG. 4 depicts a bottom plate according to an embodiment of the invention;
FIG. 5 depicts the installation of a bottom plate into a basket according to an embodiment of the invention;
FIG. 6 illustratively depicts the location relationship between the collector and the bottom plate according to the embodiment of FIG. 1 to FIG.3;
FIG. 7 illustratively depicts another embodiment regarding the location relationship between a collector and a bottom plate;
FIG. 8 illustratively depicts another embodiment regarding the location relationship between a collector and a bottom plate;
FIG. 9 illustratively depicts another embodiment regarding the location relationship between a collector and a bottom plate;
FIG. 10 illustratively depicts another embodiment regarding the location relationship between a collector and a bottom plate;
FIG. 11 depicts a cross sectional drawing with articles to be sterilized placed in the basket according to the embodiment of FIG. 1 to FIG.3;
FIG.12 is a partially enlarged drawing of FIG.11;
FIG.13 illustratively depicts the path of the steam flow entering into the feeding bottle;
FIG.14 depicts a bottle supporter of the bottom plate according to an embodiment of the invention.

### DETAILED DESCRIPTION OF EMBODIMENTS

As noted above, there is provided an improved sterilizer which addresses one or more of the existing problems.

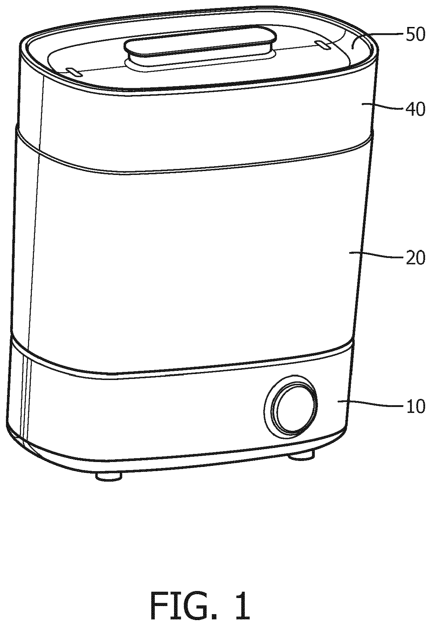

FIG. 1 shows a stereogram of a sterilizer according to an embodiment of the invention.

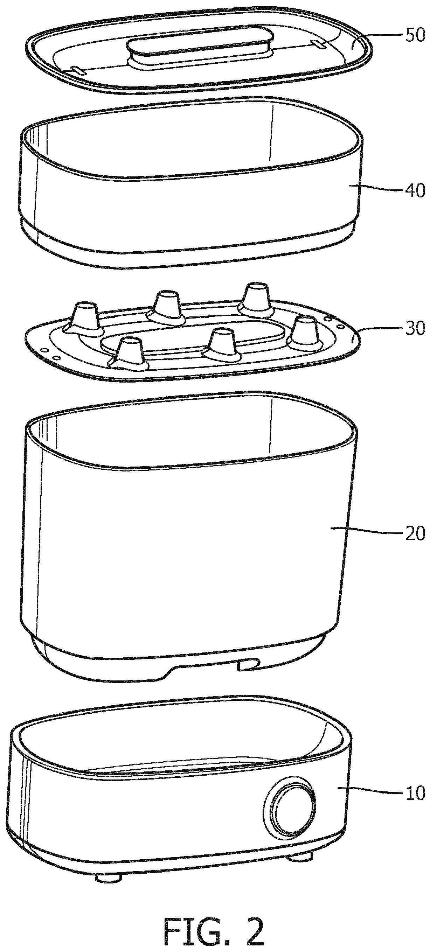

FIG.2 shows the exploded view of the sterilizer according to the embodiment of FIG. 1.

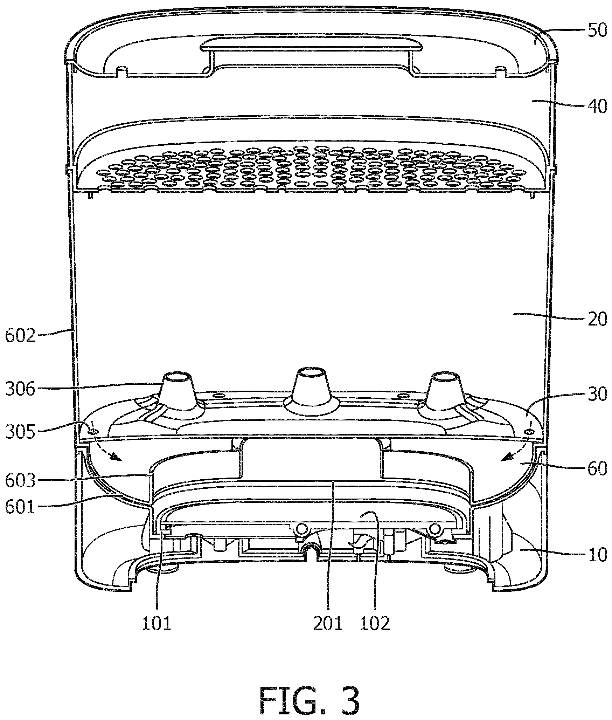

FIG.3 shows a sectional view of the sterilizer according to the embodiment of FIG.1.

It is to be understood that an element with the same function will have the same reference number in different drawings, although the detail of the implementation for the same function may be different in different embodiments.

In reference to FIG. 1 and FIG.3, according to an embodiment of the invention, the sterilizer comprises a base 10, a basket 20, an upper basket 40, and a lid 50. It is to be understood that the upper basket 40, for holding small articles such as teats, is optional.

The base 10 comprises a heating unit 101 and a water container 102. The heating unit 101 is arranged to heat water in the water container 102 to generate steam. A temperature sensing device (thermostat) may be used on the heating unit 101, so when all the water is boiled away, the heating unit switches off automatically.

The basket 20 is positioned on the base 10. The basket having an open area 201 at the bottom, which allows the generated steam to enter into the basket 20.

The bottom plate 30 is positioned in the basket 20 for holding articles to be sterilized. The articles may be for example, milk bottles, water bottles, and teats etc. The bottom plate may be detachable so that it can easily be removed for cleaning. The bottom plate 30 may also be fixed to the basket 20.

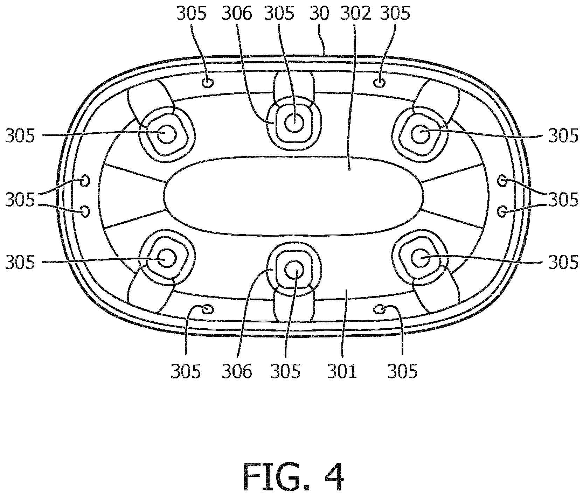

FIG.4 shows a bottom plate 30 according to an embodiment of the invention. As shown in FIG.4, The bottom plate 30 comprises a first area 301 with multiple holes 305. When the heating unit 101 heats the water in the water container 102, steam will be generated and move upwards into the basket 20 via the opening area 201 of the basket 20. The multiple holes 305 let the steam pass through the bottom plate 30 and move upwards through the basket 20 to sterilize the articles.

According to another embodiment of the invention, the multiple holes 305 may be distributed across the bottom plate, thus the whole bottom plate is the first area 301.

Alternatively, according to another embodiment of the invention, the multiple holes may only be distributed over part of the bottom plate 30. The bottom plate then comprises a second area 302, which is a solid plate without hole.

The first area 301 may be any part of the bottom plate in any shape.

For example, the first area 301 may be an annular portion or any other shape at the outer part of the bottom plate 30, and the part inside the first area 301 is the second area 302, so that the second area is in the center of the bottom plate. In this situation, the first area 301 surrounds the second area 302.

The first area 301 may be a circularportion or any other shape at the inner part of the bottom plate in the center of the bottom plate 30, and the part outside the first area 301 is the second area 302. In this situation, the second area 302 surrounds the first area 301.

Alternatively, the first area and the second area are adjacent to each other with a boundary that is a non-closed straight line or curved line. In other words, the first area 301 may also be any shape at one side of the bottom plate, and the other side is the second area 302. In this situation, the first area 301 and the second area 302 do not surround each other.

According to another embodiment of the invention, a set of holes among the multiple holes is extended upwards to form a set of hollowed pipes 306. Such hollowed pipes 306 are used to hold the feeding bottles and guide steam into the feeding bottles to sterilize them.

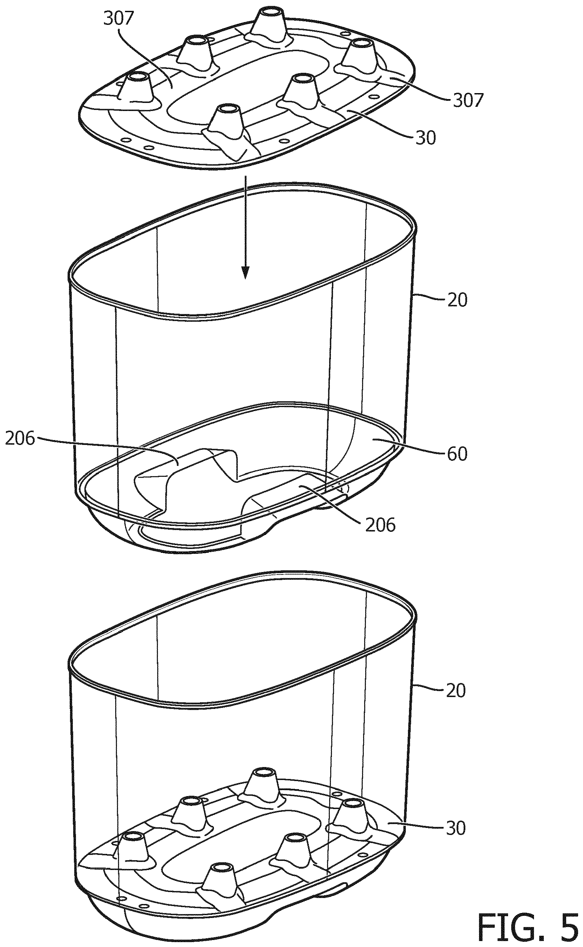

The bottom plate 30 may be detachably installed in the basket 20 or fixed to the basket 20. FIG.5 shows an example of the installation of the bottom plate 30 to the basket 20. According to the example of FIG.5, the bottom plate 30 sits on the supporters of the bottom plate 206. The drawing on the right shows the basket 20 with the bottom plate 30 in place.

Referring to FIG.3, according to an embodiment of the invention, the sterilizer further comprises a collector 60, which is positioned under the bottom plate 30 to collect fluid dripping down from the bottom plate 30. The fluid may be condensate, milk residue, or a combination of condensate and milk residue. The fluid may come from the outside and inside of the feeding bottle being sterilized, or from the upper basket 40. The fluid will flow through the holes 305 to the collector 60 as shown in FIG.3 with dotted arrow line.

The collector 60 may be fixedly or detachably attached to the basket 20, the bottom plate 30, or the base 10. The use of the collector 60 in conjunction with the bottom plate 30 stops fluid on the bottom plate from dripping into the water container.

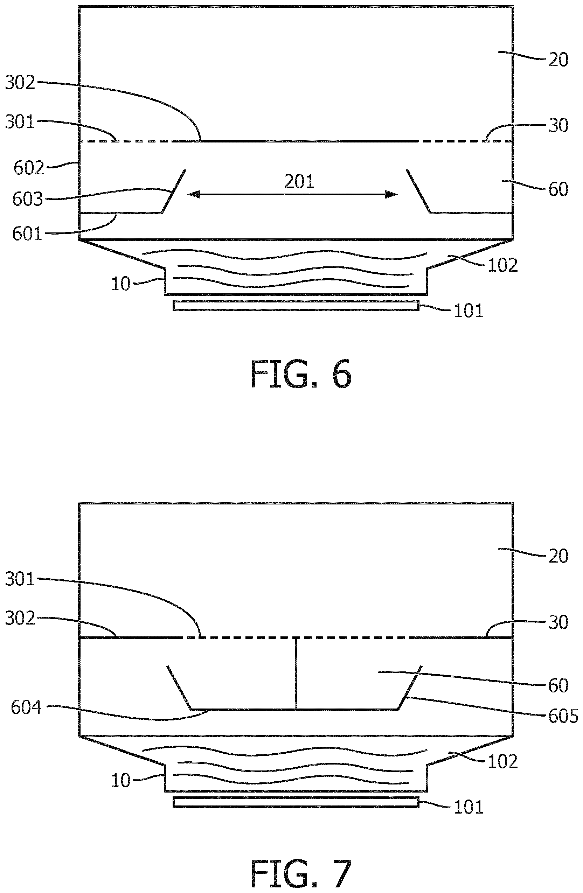

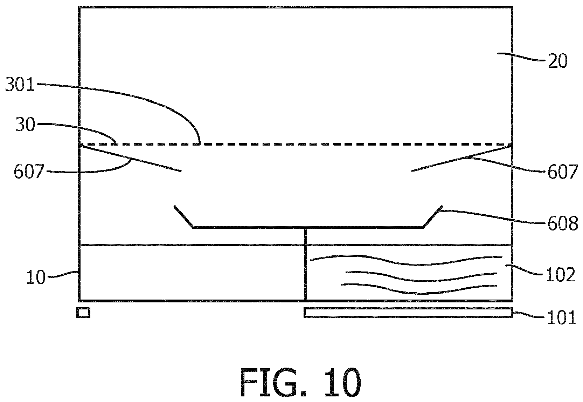

In reference to FIG.6 and FIG. 10 which illustratively showcross sections of a sterilizer with different embodiments of the collector 60 and bottom plate 30.

FIG.6 illustratively shows a position and shape of the collector 60, together with a bottom plate 30, in a basket 20, which reflects the embodiment of FIG. 1 to FIG.3. In this example, the collector 60 is in general a groove comprised of an annular bottom 601, a first sidewall 602 and a second sidewall 603. The first sidewall 602 surrounds the outer edge of the annular bottom 601 of the collector 60, and the second sidewall 603 surrounds the inner edge of the annular bottom 601 of the collector.

Advantagously, as shown in FIG.3 and FIG.6, the lower part of the sidewall of the basket 20 forms the first sidewall 602 of the collector 60, the sidewall of the basket extends inwards along the radial direction to form the bottom 601 of the collector 60. The bottom may be flat as shown in FIG.6 or it may be arc-shaped as shown in FIG.3, or any other shape.

Advantagously, the collector as shown in FIG.3 and FIG.6 may also be installed onto the bottom plate 30 or on the base 10 by attachment mechanisms, such as a snap connection, support pillar, connection pillar, or fixedly by a welded connection.

As shown in FIG.3 and FIG.6, the bottom of the collector 601 may further extend upwards to form the second sidewall 603 of the collector 60. The second sidewall 603 of the collector 60 surrounds the open area 201 of the basket 20.

The top edge of the second sidewall 603 of the collector 60 is lower than the lower surface of the bottom plate 30 to make sure there is an open space between the collector 60 and the bottom plate 30, so that steam can pass through the open space and further pass through the multiple holes of the bottom plate to reach the articles placed in the basket 20.

In the embodiments of FIG. 1 to FIG.6, the first area 301 of the bottom plate, i.e. the area with multiple holes is at the outer part of the bottom plate and the second area 302 is surrounded by the first area 301, the horizontal projection of the collector 60 covers the horizontal projection of the first area 301 such that the fluid dripping from the multiple holes flows into the collector 60. In other words, the position of the collector vertically corresponds to the position of the first area 301, and the horizontal projection of the collector is equal to or larger than the horizontal projection of the first area 301 in size. It is to be understood that the term "cover" in the context of the invention means the horizontal projection of the first area 301 is contained in the horizontal projection of the collector 60.

FIG.7 to FIG. 10 shows some other embodiments in the locational relationship of the collector 60 and bottom plate 30. In these examples, the collector 60 comprises a bottom 604 and a sidewall 605 surrounding the bottom 604. In other words, the collector 60, in this example, only has only one sidewall, e.g. like a bowl or any other shape. The top edge of the sidewall 605 of the collector 60 is lower than the lower surface of the bottom plate 30 to make sure there is open space between the collector 60 and the bottom plate let steam pass through.

Similar to the aforementioned embodiments, the collector 60 of FIG.7 to FIG. 10 may also be installed to a proper place at the base 10, the basket 20 or the bottom plate 30.

The proper place depends on the location of first area 301 of the bottom plate 30, so long as the horizontal projection of the collector 60 covers the horizontal projection of the first area 301.

In the example of FIG.7, the first area 301 is in the center of the bottom plate 30, and thus the collector 60 is positioned under the first area 301 to collect the fluid.

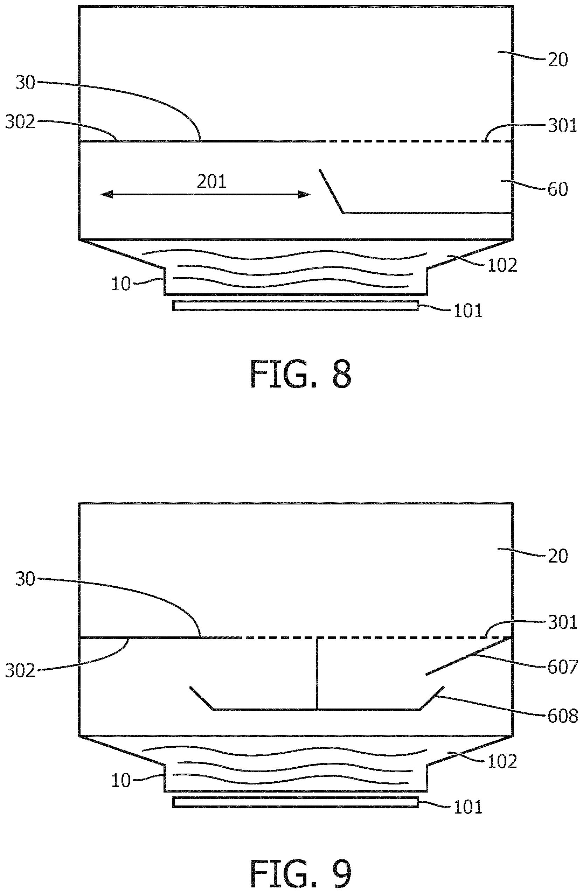

In the examples of FIG.8 and FIG.9, the first area 301 is at one side of the bottom plate 30, and the collector 60 is correspondingly under the first area 301.

FIG.9 and FIG. 10, show other embodiments of a collector 60, which comprises two separate parts 607 and 608. The first part 607 may be an inclined plate to guide the flow of fluid into the second part 608, which is a bowl shaped container.

In the examples of FIG.6 to FIG.9, the second area 302 does not have any hole, and the horizontal projection of the second area covers the horizontal projection of the open area 201 at the bottom of the basket 20. Because the second area is a solid area without a hole, when positioned to cover the open area of the basket, fluid cannot flow out of the basket 20, thus, no fluid can drip down into the water container 102.

In the example of FIG. 10, the bottom plate 30 does not have a second area 302, and multiple holes are distributed across the whole of the bottom plate 30.

The shape of the collector 60 may correspond to the shape of the first area 301 of the bottom plate 30. The shape of the collector and the first area may also be different as long as the horizontal projection of the collector 60 covers the horizontal projection of the first area 301.

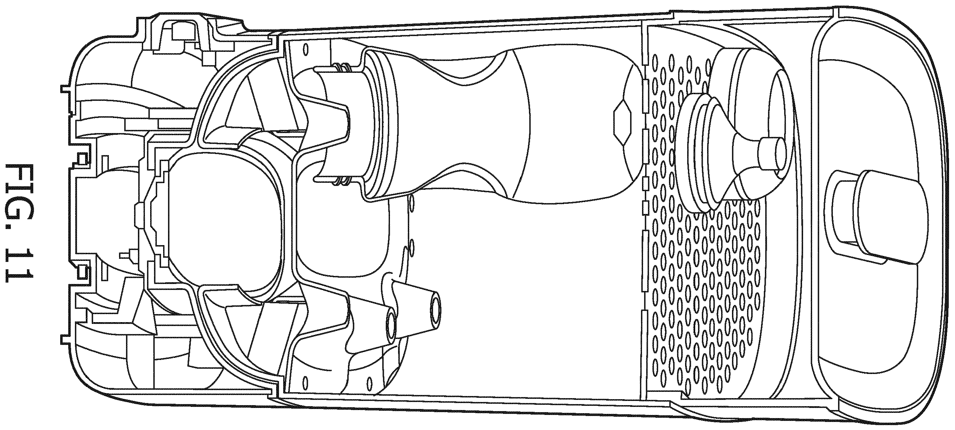

FIG.11 shows a cross sectional drawing with articles to be sterilized placed in the basket according to the embodiment of FIG. 1 to FIG.3.

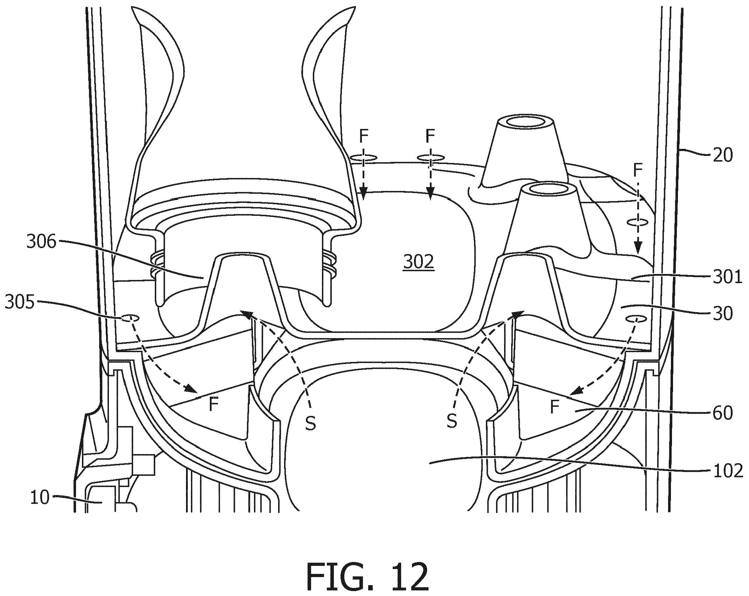

FIG.12 is a partially enlarged drawing of FIG.11. In reference to FIG.11 and FIG.12, a feeding bottle is placed on a hollowed pipes306. The steam is generated in the water container 102 and rises up from the water container 102 to enter into the feeding bottle via the hollowed pipe 306. The flow of the steam is indicated by the dotted arrow lines S. The fluid flows out through each hole 305. The fluid flow is indicated by the dotted lines F.

During and after the sterilizing process, condensate fluid flows out of the feeding bottle. There are no holes in the second area 302, thus the fluid cannot drip back into the water container. The fluid can only flow through the holes 305 in the first area 301. In this way, the fluid is directed into the collector 60. The flow of the fluid is indicated by the arrows F.

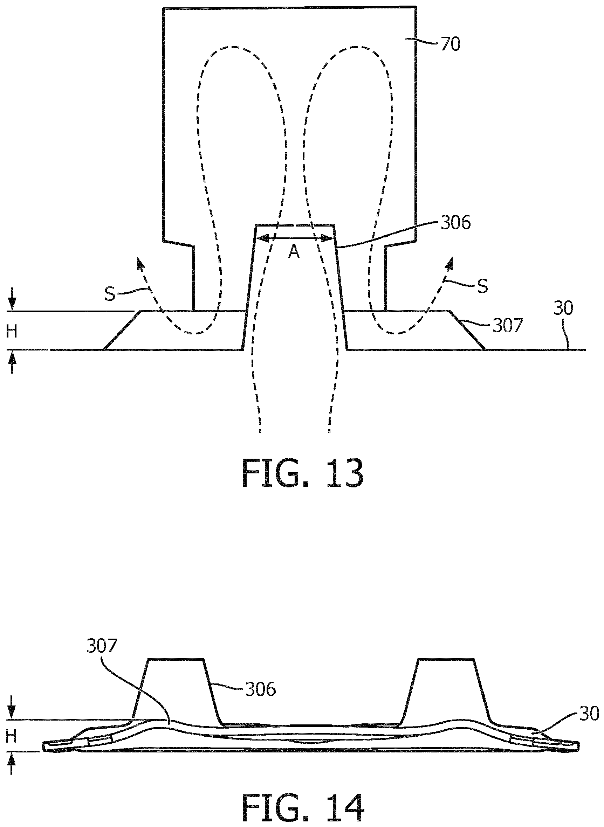

FIG.13 illustratively shows the flow of the steam flow S as it enters into sterilizes, and then escapes from the feeding bottle 70. The feeding bottle 70 is put on at least two bottle holders 307. The bottle holders 307, create the necessary space for the flow of steam from the hollowed pipe 306, through the feeding bottle 70, and then back into main chamber of the basket.

To improve the efficiency of the steam flow and sterialization, according to one embodiment of the invention, the total area of the outlets of the hollowed pipes 306 is larger than the total area of the multiple holes 305, this makes more steam go into the inside of the feeding bottles.

According to another embodiment of the invention, steam might be misallocated to a hollowed pipe 306 that is not covered by a feeding bottle, because without the feeding bottle there is less resistance to the steam. The concern is that this is a waste of steam and that less steam will then be available to move through another hollow pipe 306 that is covered by a feeding bottle.. This concern is overcome by the diameter of the outlet of the hollowed pipe indicated by the arrowed line A in FIG.13 and height of the bottle supporters. In this emodiment of the invention, The diameter of the outlet of each of the hollowed pipes is designed to be smaller than 13 millimeters, and the height H of the bottle supporter 307 from the basic plane of the bottom plate 30 is designed to be more than 4 millimeters. By this way the flow resistance of the hollowed pipe 306 leading into the feeding bottle is substantially larger than the resistance for steam to flow out of the feeding bottle. Thus, in an instance where for example 5 out of 6 hollowed pipes are each covered with a feeding bottle, the flow resistance of the one uncovered hollowed pipe is not substantially reduced and it has no greater volume of steam flowing through it than the 5 other feeding bottles.

When the sterilizer, in accordance to the invention, also includes a drying cycle, which uses a fan to provide a heated air flow to dry sterilized feeding bottles, the aforementioned size of the hollowed pipes, multiple holes, and the height of the bottle supporters may also provide a better air flow to dry the feeding bottles.

As shown in FIG. 14, according to one embodiment of the invention, the bottle supporter 307 may be formed integrally in the bottom plate 30 as a shape that curves upwards on the surface.

Variations to the disclosed embodiments can be understood and effected by those skilled in the art of practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A sterilizer comprising:
- a base (10) comprising a heating unit (101) and a water container (102), the heating unit is arranged to heat water in the water container to generate steam;
- a basket (20) positioned on the base (10), the basket having an open area (201) at the bottom to let steam enter into the basket;
- a bottom plate (30) positioned in the basket (20) for holding articles to be sterilized;
- a collector (60) positioned under the bottom plate for collecting fluid dripping down from the bottom plate.

2. The sterilizer according to claim 1, wherein the collector (60) is fixed or detachably attached to the basket (20), the bottom plate (30) or the base (10).

3. The sterilizer according to claim 2, the collector (60) being a groove comprising an annular bottom (601), a first sidewall (602) and a second sidewall (603), wherein the first sidewall (602) surrounds the outer edge of the annular bottom (601) of the collector (60), and the second sidewall (603) surrounds the inner edge of the annular bottom of the collector (60).

4. The sterilizer according to claim 3, wherein the sidewall of the basket forms the first sidewall (602) of the collector (60), the sidewall of the basket extends inwards along the radial direction to form the bottom (601) of the collector and further extends upwards to form the second sidewall (603) of the collector.

5. The sterilizer according to claim 4, wherein the second sidewall (603) of the collector surrounds the open area (201) of the basket (20).

6. The sterilizer according to any one of claims 3 to 5, wherein the top edge of the second sidewall (603) of the collector is lower than the lower surface of the bottom plate.

7. The sterilizer according to claim 2, wherein the collector comprises a second bottom (604) and a third sidewall (605) surrounding the second bottom (604).

8. The sterilizer according to claim 7, wherein the top edge of the third sidewall (605) of the collector is lower than the lower surface of the bottom plate.

9. The sterilizer according to any one of claims 1 to 8, the bottom plate being detachably or fixedly positioned in the basket, the bottom plate comprising a first area (301) with multiple holes (305) allowing steam from the water container (102) to pass through, wherein the horizontal projection of the collector (60) covers the horizontal projection of the first area (301) of the bottom plate (30).

10. The sterilizer according to claim 9, the bottom plate further comprising a second area (302) that is a solid plate without holes, wherein the horizontal projection of the second area (302) covers the horizontal projection of the open area (201) of the basket (20).

11. The sterilizer according to claim 10, wherein the second area (302) of the bottom plate (20) is in the center of the bottom plate (20) and is surrounded by the first area (301) of the bottom plate (20), or the first area (301) is in the center of the bottom plate and is surrounded by the second area (302), or the first area (301) and the second area (302) of the bottom plate (30) are adjacent to each other with a boundary being a non-closed straight line or curved line.

12. The sterilizer according to any one of claims 9 to 11, wherein a set of holes among the multiple holes (305) is extended upwards to form a set of hollowed pipes (306).

13. The sterilizer according to claim 12, wherein the total area of the outlet of the set of hollowed pipes (306) is larger than the total area of the multiple holes (305) without hollowed pipes.

14. A basket (20) for use in a sterilizer which sterilizes articles using steam, the basket having an open area (201) at the bottom to let steam into the basket, a bottom plate (30) positioned in the basket for holding articles to be sterilized, wherein the basket (20) comprises a collector (60) for collecting fluid dripping down from the bottom plate.

15. A bottom plate (30) for use in a sterilizer which sterilizes articles using steam, the bottom plate being used for holding articles to be sterilized, the bottom plate (30) being positioned in a basket (20), wherein the bottom plate comprises a collector (60) positioned under the bottom plate for collecting fluid dripping down from the bottom plate.
